# EUROPEAN PATENT APPLICATION

(11) **EP 3 103 457 A1**
(43) Date of publication of application: **14.12.2016**
(21) Application number: 14881871.9
(22) Date of filing: 17.04.2014
(51) Int. Cl.: A61K 31/7004, A61P 3/04

(54) **SATIETY MAINTAINING AGENT AND METHOD FOR MAINTAINING FEELING OF SATISFACTION**

(30) Priority: 05.02.2014 JP 2014019965; 28.02.2014 JP 2014037956
(71) Applicant: Jichi Medical University, Shimotsuke-shi, Tochigi 329-0498 (JP); Matsutani Chemical Industry Co., Ltd., Itami-shi, Hyogo 664-8508 (JP)
(72) Inventor: YADA, Toshihiko, Shimotsuke-shi Tochigi 329-0498 (JP); IWASAKI, Yusaku, Shimotsuke-shi Tochigi 329-0498 (JP); KIMURA, Tomonori, Itami-shi Hyogo 664-8508 (JP); OHKUMA, Kazuhiro, Itami-shi Hyogo 664-8508 (JP)
(74) Representative: TBK
(86) International application number: PCT/JP2014/060908
(87) International publication number: WO 2015/118699

(57) **Abstract**

Object: To provide an agent for sustaining satiety or a method for sustaining satiety, with which an individual's food intake is reduced by sustaining satiety, and as a result, pathological conditions such as obesity due to overeating can be prevented and improved.

Means for Resolution: An agent for sustaining satiety containing D-psicose as an active ingredient and a method for sustaining satiety by the ingestion of D-psicose.

Advantageous Effect: Satiety can be sustained although the increase in blood glucose level after eating is suppressed. A means for sustaining satiety which is useful for those who are anxious about their blood glucose level and those who are health conscious can be provided.

## Description

### Technical Field

The present invention relates to an agent for sustaining satiety or a method for sustaining satiety, with which satiety can be sustained by the ingestion of D-psicose in the body, and relates to an agent for sustaining satiety or a method for sustaining satiety, with which an individual's food intake, that is, energy intake is reduced by sustaining satiety, and as a result, pathological conditions such as obesity due to overeating can be prevented and improved.

### Background Art

It is very important to prevent and improve obesity for health maintenance and promotion. Obesity is one of the pathological conditions occurring as a result of excess accumulation of carbohydrates and fats due to excess intake of foods as triglycerides in subcutaneous adipose tissues and tissues surrounding organs. Among these, the increased visceral adipose tissue synthesizes and secretes adipocytokines such as TNF-α to adversely affect the carbohydrate metabolism or the lipid metabolism, and therefore, obesity due to visceral adipose tissue accumulation is seen as a more serious problem. If the condition of visceral adipose tissue accumulation, hyperglycemia, or hyperlipidemia lasts for a long time due to abnormal metabolism, a risk of progression to a disease such as diabetes, arteriosclerosis, hypertension, or a heart disease is increased, and thus, such a condition is extremely dangerous.

In order to prevent such a disease, it is important to prevent obesity, and therefore, it is apparent that the restriction of excess energy intake is the best means. However, when the energy intake is restricted, that is, when food intake is restricted, persistent hunger lowers the vitality of life. When hunger cannot be tolerated and the food restriction is stopped, food intake is increased instead as compared with before. In this manner, it is very difficult to constantly internalize food restriction as a habit of life, and therefore, a method for sustaining satiety for a long time has been strongly demanded.

The mechanism in which we feel satiety when eating is that the stomach wall is expanded and the blood glucose level increases. There has been reported that the expansion of the stomach wall stimulates the vagal afferent nerves distributed in the stomach, or the increase in blood glucose level due to D-glucose generated by the degradation of carbohydrates in foods stimulates the satiety center in the hypothalamus of the brain, and thus, we feel satiety (NPL 1 to NPL 4).

On the other hand, however, the intake of a food or a substance which hardly increases the blood glucose level is considered to be effective in the prevention and the treatment of carbohydrate metabolism disorders such as diabetes. Therefore, when a patient suffering from such a disorder wants to feel satiety, it is considered that the patient does not ingest a food or the like which increases the blood glucose level, but adopts a method of ingesting a food or the like which physically expands the stomach wall. Examples of such a food include polysaccharides which have almost no calories, but retain a lot of water and are gelled such as agar.

As conventional agents for sustaining satiety, there are so many agents utilizing a polysaccharide as exemplified above. For example, a composition for suppressing hunger composed mainly of agar (PTL 1), a complex containing whole grains and a hydrocolloid (PTL 2), rice supplemented with insoluble crystalline cellulose, characterized by adding or coating of insoluble crystalline cellulose (PTL 3), and a method for causing the response to hypoglycemia in a diabetic patient including administering pullulan to a diabetic patient (PTL 4) are all for sustaining satiety by utilizing a polysaccharide having almost no calories. Other than these, agents for utilizing a protein or a peptide such as an agent for sustaining satiety containing polyglutamic acid as an active ingredient (PTL 5), a composition for inducing satiety containing fermented whey as an active ingredient (PTL 6), and a food composition containing an edible phosphoprotein and a specific metal salt (PTL 7) have been disclosed. Further, an agent utilizing an amylase inhibitor (PTL 8) is also known.

However, when a polysaccharide which retains a lot of water is utilized, the viscosity thereof is problematic and makes it hard to use. Insoluble crystalline cellulose is insoluble in water, and therefore, the utilization thereof is limited to a specific subject such as a solid. Further, a peptide, or the above-described amylase inhibitor is derived from a protein, and therefore has an unfavorable flavor, and moreover has a problem that it cannot be used for a patient who has allergic symptoms against such a substance.

Further, as an appetite suppressant having an anti-obesity activity, there is known a pharmaceutical composition which directly acts on a neurotransmitter in the brain (PTL 9) . However, such an agent acts on the entire brain as a serotonin agonist or a dopamine agonist, and therefore not only suppresses appetite, but also has a problem of having serious side effects such as abnormal blood pressure, gastrointestinal disorders, and neuropsychiatric symptoms.

### Citation List

### Patent Literature

PTL 1: Japanese Patent No. 4074325
PTL 2: JP-A-2012-214785
PTL 3: JP-A-2008-73026
PTL 4: JP-T-2005-529944
PTL 5: JP-A-2013-129612
PTL 6: JP-A-2011-239774
PTL 7: JP-A-2010-94085
PTL 8: JP-A-9-194392
PTL 9: JP-A-11-228447
PTL 10: WO 2008/142860

### Non Patent Literature

NPL 1: Juhasz A, et al. Orv Hetil. 148: 1827-1836 (2007)
NPL 2: Sobocki J, et al. J Physiol Pharmacol. 56: 27-33 (2005)
NPL 3: Li Y. Curr Med Chem. 14: 2554-2563 (2007)
NPL 4: Mayer J. Ann N Y Acad Sci. 63: 15-43 (1955)
NPL 5: Matsuo T, et al. J Nutr Sci Vitaminol. 48: 77-80 (2002)
NPL 6: Hayashi N, et al. Biosci Biotechnol Biochem. 74: 510-9 (2010)
NPL 7: Iida T, et al. J Nutr Sci Vitaminol. 54: 511-4 (2008)
NPL 8: Matsuo T, et al. J Nutr Sci Vitaminol. 30: 55-65 (2001)
NPL 9: Hishiike T, et al. J Agric Food Chem. 61: 7381-6 (2013)
NPL 10: Koichi N, et al. Proceedings of the second symposium of international society of rare sugars. 341-5 (2004)
NPL 11: Flint A, et al. Int J Obes Relat Metab Disord. 24: 38-48 (2000)

### Summary of Invention

### Technical Problem

Conventionally, it has been reported that D-psicose has no calories (NPL 5), and has an effect of suppressing the increase in blood glucose level after eating (PTL 1, NPL 6, and NPL 7), an anti-obesity effect (NPL 8), and so on, and particularly with respect to the blood glucose level, it has been confirmed that the increase in blood glucose level after eating is suppressed by ingesting 5 g of D-psicose along with a regular meal in humans (NPL 6 and NPL 7). Further, it has been known that even if D-psicose is ingested alone, the blood glucose level does not increase (NPL 7). It has been also revealed that the absorption of D-psicose in the small intestine is slower as compared with D-glucose (grape sugar) and D-fructose (fruit sugar) (NPL 9). Further, it has been known that D-psicose does not directly act on the brain (NPL 10).

However, there has been no report so far that the ingestion of D-psicose sustains satiety, and the ingestion of D-psicose during fasting reduces appetite and food intake thereafter. On the other hand, it has been known that the increase in blood glucose level or the expansion of the stomach by a highly viscous substance is important for inducing or sustaining satiety. However, D-psicose does not increase the blood glucose level and does not substantially show viscosity, and therefore, a prediction that D-psicose sustains satiety has not been made.

An object of the present invention is to provide an agent for sustaining satiety, which sustains satiety although it suppresses the blood glucose level after eating without directly acting on the brain, and also can be ingested without causing any discomfort with respect to texture or flavor, or a method for sustaining satiety.

### Solution to Problem

As a result of intensive studies, the present inventors found that a specific dose of D-psicose has an effect of sustaining satiety after ingesting a meal, and thus completed the present invention.

A first invention of the present invention is an agent for sustaining satiety characterized by containing D-psicose as an active ingredient.

A second invention of the present invention is characterized by being an agent for sustaining satiety by transmitting the oral ingestion of D-psicose to the brain via the vagal afferent nerves in the first invention.

A third invention of the present invention is characterized in that D-psicose is contained such that D-psicose is ingested in an amount of 0.07 g to 3 g per kilogram of body weight with one meal in the first invention.

A fourth invention of the present invention is characterized in that the agent is used between one hour before eating and immediately before eating in the first invention.

A fifth invention of the present invention is characterized in that the agent is used for an individual who needs suppression of the increase in blood glucose level after eating or an obese individual in the first invention.

A sixth invention of the present invention is characterized in that the agent is used for producing a diet food or drink in the first invention.

A seventh invention of the present invention is a method for sustaining satiety characterized by including a step of making a subject to ingest D-psicose or administering D-psicose to a subject.

An eighth invention of the present invention is characterized in that the step of ingesting or administering D-psicose includes ingesting or administering D-psicose in an amount of 0.07 g to 3.0 g per kilogram of body weight with one meal in the seventh invention.

A ninth invention of the present invention is characterized in that the step of ingesting or administering D-psicose is provided between one hour before eating and immediately before eating in the seventh invention.

A tenth invention of the present invention is characterized in that the step of ingesting or administering D-psicose is provided at every meal in the seventh invention.

An eleventh invention of the present invention is characterized in that the step of ingesting or administering D-psicose is provided once daily before breakfast in the seventh invention.

A twelfth invention of the present invention is characterized in that the step of ingesting or administering D-psicose is performed through oral ingestion or oral administration of D-psicose, and the oral ingestion or oral administration thereof is transmitted to the brain via the vagal afferent nerves, whereby satiety is sustained in the seventh invention.

A thirteenth invention of the present invention is characterized in that the subject is an individual who needs suppression of the increase in blood glucose level after eating or an obese individual in the seventh invention.

### Advantageous Effects of Invention

The present invention can provide an agent for sustaining satiety or a method for sustaining satiety, with which sustained satiety is provided. The agent for sustaining satiety or the method for sustaining satiety of the present invention provides satiety in the case of ingestion thereof, and also the satiety lasts for a given period of time.

The agent for sustaining satiety or the method for sustaining satiety of the present invention not only does not increase the blood glucose level by a single ingestion, but also suppresses the increase in blood glucose level after eating in the case of ingestion before eating, and moreover has an effect of sustaining satiety for a given period of time although it suppresses the increase in blood glucose level after eating. Therefore, calorie restriction can be effectively carried out without any regard to food intake restriction, and as a result, the prevention and improvement of lifestyle-related diseases such as hypertension, hyperlipidemia, and diabetes can be expected. Further, the agent for sustaining satiety of the present invention does not directly act on the brain, but transmits the information to a localized brain region via the vagal afferent nerves distributed in the periphery to sustain satiety, and therefore, the agent does not have serious side effects.

Accordingly, by using the agent for sustaining satiety or the method for sustaining satiety of the present invention, overeating can be avoided, and therefore, the agent or the method is useful for preventing or improving obesity, lifestyle diseases, and so on. Further, by using the agent for sustaining satiety of the present invention, implementation of diet is facilitated. That is, a diet food or drink capable of sustaining satiety by ingesting the agent for sustaining satiety of the present invention can be provided.

Further, the present invention has been demonstrated to exhibit the effect of sustaining human satiety, reducing hunger, and reducing appetite, and therefore can contribute to health promotion such as prevention of obesity due to human overeating and reduction in incidence rate of lifestyle-related diseases and adult diseases.

### Brief Description of Drawings

[Fig. 1] Fig. 1 shows the transition of cumulative food intake (relative value (%)) when D-psicose was orally administered by gavage to mice.
[Fig. 2] Fig. 2 shows the transition of cumulative food intake (relative value (%)) when D-psicose was intraperitoneally administered to mice.
[Fig. 3] Fig. 3 shows the transition of cumulative food intake (kcal) when D-glucose was orally administered by gavage to mice.
[Fig. 4] Fig. 4 shows the results of a conditioned taste aversion test with respect to the oral administration of D-psicose using mice.
[Fig. 5] Fig. 5 shows the expression level of c-Fos (a marker of neuronal activation) in the nodose ganglion and in the medullary nucleus tractus solitarius of mice after orally administering D-psicose by gavage.
[Fig. 6] Fig. 6 shows the transition of cumulative liquid food intake (g) after orally administering D-psicose by gavage to the mice received sham operation or subdiaphragmatic vagotomy.
[Fig. 7] Fig. 7 shows the transition of satiety after eating when D-psicose was ingested by humans.
[Fig. 8] Fig. 8 shows the transition of hunger after eating when D-psicose was ingested by humans.
[Fig. 9] Fig. 9 shows the transition of appetite after eating when D-psicose was ingested by humans.

### Description of Embodiments

The present invention relates to an agent for sustaining satiety containing D-psicose as an active ingredient and a method for sustaining satiety, and contributes to the maintenance of healthy bodies by preventing excess food intake.

The effect of sustaining satiety of D-psicose does not result from discomfort or disgust after ingesting D-psicose. Further, D-psicose does not directly act on the feeding center of the brain, but acts on the brain via the vagal afferent nerves in the visceral sensory nerve widely distributed in the peripheral organs, whereby satiety is induced and sustained. Since the effect of sustaining satiety, reducing hunger, and reducing appetite is exhibited in humans who ingest D-psicose, and therefore, the present invention is useful for preventing human health hazard due to overeating.

The term "satiety" refers to a state where satiety obtained by a given amount of a meal is sustained, a state where hunger is suppressed, or a state where appetite is suppressed. As a method for determining this state, a questionnaire prepared by modifying a Visual Analog Scales (VAS) method (NPL 11) is used. In particular, determination is made according to the item of level of desire to eat. Further, in the present invention, the phrase "sustaining satiety" indicates that the above-described satiety is sustained for at least 2 hours or more, preferably 3 hours or more, more preferably 4 hours or more.

D-psicose to be used in the present invention is one of the monosaccharides (rare sugars) whose abundance in nature is low, has a history of use in a food, and is a hexose (C₆H₁₂O₆). The degree of sweetness of D-psicose is about 70% of that of sugar, and D-psicose has a refined and refreshing sweetness similar to D-fructose, but has almost no calories. As the production of D-psicose, a production method in which D-psicose is obtained by treating D-fructose with an epimerase enzyme (PTL 10) and the like are known, however, an extract from a natural product such as Itea plant may be used, or D-psicose contained in a natural product may be used after extraction and purification.

The agent for sustaining satiety containing D-psicose as an active ingredient of the present invention can be used in a general sweetener or a food or drink. Further, it can be used as a starting material for any preparations such as pharmaceutical products, quasi drugs, or feedstuff. In addition, the food or drink includes functional foods and drinks such as beauty foods and drinks, foods and drinks for sick people, and foods and drinks for specified health use, which are prepared on the concept of sustaining satiety or suppressing hunger, and if necessary, have a label describing the concept.

The above-described foods and drinks are not particularly limited, and correspond to all sorts of products, and the agent can be used in, for example, confectionery such as pudding, jelly, candy, chocolate, bread, cakes, cookies, and steamed bean-jam buns; egg products such as custard cream; drinks such as functional drinks, lactic acid drinks, fruit juice drinks, and carbonated drinks; favorite products such as tea and instant coffee; dairy products such as ice cream, yogurt, and cheese; pastes such as flour paste and fruit preserved in syrup; animal meat products such as hams, sausages, and bacons; processed marine products such as fish hams and fish sausages; seasonings such as soy sauce, Worcestershire sauce, and dressings; and the like.

Further, the agent can also be used as a functional food or a nutritional supplement food such as a liquid food, an elemental nutrition food, a drink nutrition food, or an enteral nutrition food. The form thereof is not particularly limited, however, for example, in the case of a sport drink, in order to enhance the nutritional balance and the flavor, it is also possible to blend a nutritional additive such as an amino acid, a vitamin, or a mineral, a composition, a flavoring material, a dye, or the like.

As the ingestion form of the pharmaceutical product and the quasi drug, other than the single use of D-psicose, D-psicose can be used in combination with another material in the form of a tablet, a capsule, a granule, a powder, a liquid, or the like. The administration form is not limited to oral administration, and parenteral administration by an injection, a patch, a spray, or the like can also be adopted. Further, such a pharmaceutical preparation can be prepared by combining an appropriate additive such as a general excipient, stabilizer, preservative, binder, or disintegrant. Among the above-described administration form of the pharmaceutical product, a preferred form is oral administration, and preferably, the pharmaceutical product is administered to an individual who needs suppression of the increase in blood glucose level after eating or an obese individual.

Examples of the feedstuff include feedstuff for domestic animals, domestic fowls, and pets, and for example, feedstuff for small animals to be used for rabbits, rats, mice, etc., pet foods for dogs, cats, small birds, etc., and feedstuff for domestic animals such as cattle and pigs. In particular, in order to prevent dogs, cats, etc. kept at home from being obese due to overeating and lack of exercise, it is effective to give the agent for sustaining satiety of the present invention along with feed.

The ingestion amount of D-psicose in the agent for sustaining satiety containing D-psicose as an active ingredient or the method for sustaining satiety of the present invention is not particularly limited as long as its effect is obtained, however, D-psicose is preferably ingested in an amount of 0.07 g to 3.0 g per kilogram of body weight with one meal. The amount range is more preferably from 0.1 to 2.0 g, and further more preferably from 0.3 to 1.5 g. The ingestion amount varies depending on the health conditions, body weight, gender, or age of the subject, or other conditions, however, for example, the amount of D-psicose which can be ingested at one time per adult (60 kg) is preferably 30 g in consideration of a laxative property. Further, the ingestion timing is preferably before eating, more preferably within 1 hour before eating, further more preferably within 15 to 30 minutes before eating.

When the agent for sustaining satiety of the present invention is ingested, the effect is exhibited in about 15 minutes after the ingestion, and therefore, by sustaining satiety thereafter, appetite is reduced and food intake is reduced. The duration of satiety after the ingestion varies depending on the conditions, however, it was confirmed that the duration is from 6 hours to 12 hours after the ingestion, and those who ingested the agent return to the state before the ingestion after 24 hours. In order to reliably sustain satiety, it is preferred to ingest the agent for sustaining satiety of the present invention before each meal. However, the agent for sustaining satiety of the present invention exhibits its effect of sustaining satiety for about 12 hours, and therefore, by ingesting the agent once before breakfast, satiety is obtained when eating breakfast, lunch, and supper, and thus, food intake can also be restricted.

The subject who ingests the agent for sustaining satiety of the present invention is not particularly limited, and may be a healthy subject or a subject who wants to prevent or improve lifestyle-related diseases such as hypertension, hyperlipidemia, and diabetes, and may be any subject who wants to restrict the intake of foods.

According to the previous studies, it is known that D-psicose which is the active ingredient of the agent for sustaining satiety of the present invention exhibits an anti-oxidative activity, a cryoprotective activity, an activity of suppressing the increase in blood glucose level after eating, an antiarteriosclerotic activity, an activity of suppressing the degeneration of pancreatic β cells, and an activity of suppressing fat accumulation, and also does not have side effects on human body, and therefore is a safe substance. Accordingly, by utilizing these activities, it is possible to simultaneously treat lifestyle-related diseases such as hypertension, hyperlipidemia, and diabetes, and also the subject who ingests the agent is not limited. Further, since D-psicose is a substance to be used as a sweetener, there is no difficulty in oral ingestion, and moreover, even if it is added to food products, it is possible to eat the food products without causing any discomfort.

The application of the agent for sustaining satiety of the present invention is not limited to humans, and the agent can exhibits its effect in any mammals.

The agent for sustaining satiety of the present invention is used also for producing a diet food or drink and is added to a diet food such that D-psicose is ingested in an amount of 0.07 g to 3 g per kilogram of body weight with one meal.

The present invention will be described in more detail with reference to Examples, however, the invention is by no means limited to the Examples.

### Example 1

The activity of reducing food intake by D-psicose in fasted mice was confirmed by the following test.

### <Test Method>

As the experimental animal, C57BL/6J male mice were used. The mice were acclimated to the environment by preliminary rearing for 1 week or more in individual cages. After fasting the mice for 16 hours from 18:00 p.m. on the previous day of the experiment, each test solution was orally administered by gavage or intraperitoneally administered to the mice. In each administration route, physiological saline (10 mL/kg) was administered in a control group, and D-psicose (0.3 g/kg, 1 g/kg, or 3 g/kg) was administered in a test group. The concentration of the respective test solutions at this time was 0.3%, 10%, and 30%, respectively. Each solution was administered at 9:45 a.m., and from 10:00 a.m., the mice were allowed free access to CE-2 feed (a standard mouse feed with a good nutritional balance manufactured by CLEA Japan, Inc.), and the food intake was measured over time after 0.5 hours, 1 hour, 2 hours, 3 hours, 6 hours, and 24 hours.

Incidentally, as a comparative example, an experiment of oral administration by gavage of D-glucose (1 g/kg and 3 g/kg), which has a sweetness equivalent to D-psicose and has a similar structure, but is metabolized to contribute to energy production, was performed in the same manner.

### <Test Results>

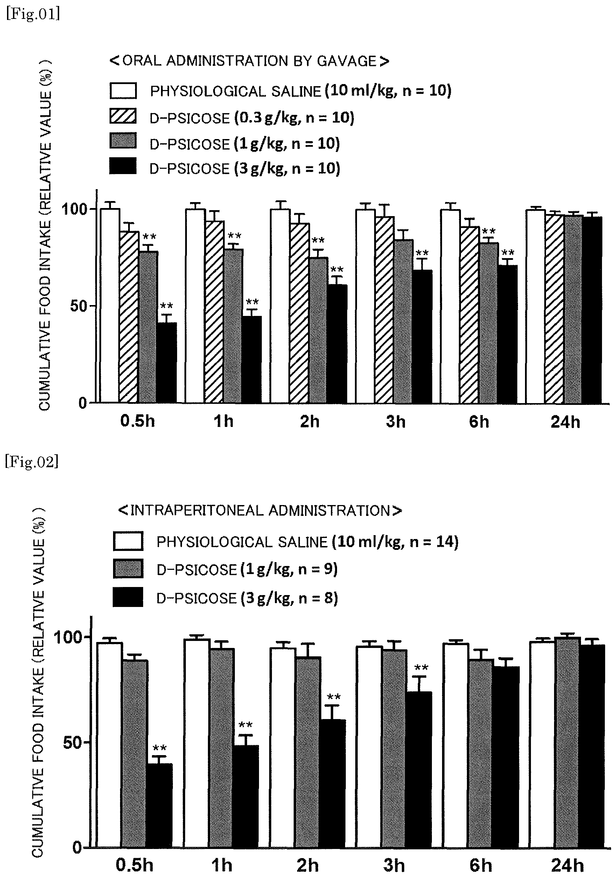

The results obtained by plotting the elapsed time after administration on the abscissa, and the cumulative food intake (relative value (%)) at each elapsed time on the ordinate are shown in Fig. 1 (oral administration by gavage) and Fig. 2 (intraperitoneal administration). Incidentally, the cumulative food intake (relative value (%)) is expressed as the ratio of the cumulative food intake in each group with the average food intake in the physiological saline group at each time in each experiment performed taken as 100%. The obtained results are expressed as a mean and a standard error. A statistical test was performed by a one-way analysis of variance, and if a significant difference was detected, a Dunnett's test in comparison with the control group was performed. The significance level for the test was set to less than 5% on both sides, and the data was marked with * when the P value was less than 5%, and the data was marked with ** when the P value was less than 1%.

As a result, when D-psicose was orally administered by gavage to the mice which were fasted overnight and were in a hungry state, a change in food intake was not observed in any time zones after the administration in the 0.3 g/kg administration group. However, in the 1 g/kg D-psicose administration group and the 3 g/kg D-psicose administration group, the food intake was significantly decreased in the period between 0.5 and 6 hours after the administration (Fig. 1). On the other hand, in the case of intraperitoneal administration, in the 1 g/kg D-psicose administration group, there was no effect on food intake, and in the 3 g/kg D-psicose administration group, the food intake was significantly decreased in the period between 0.5 and 6 hours after the administration (Fig. 2).

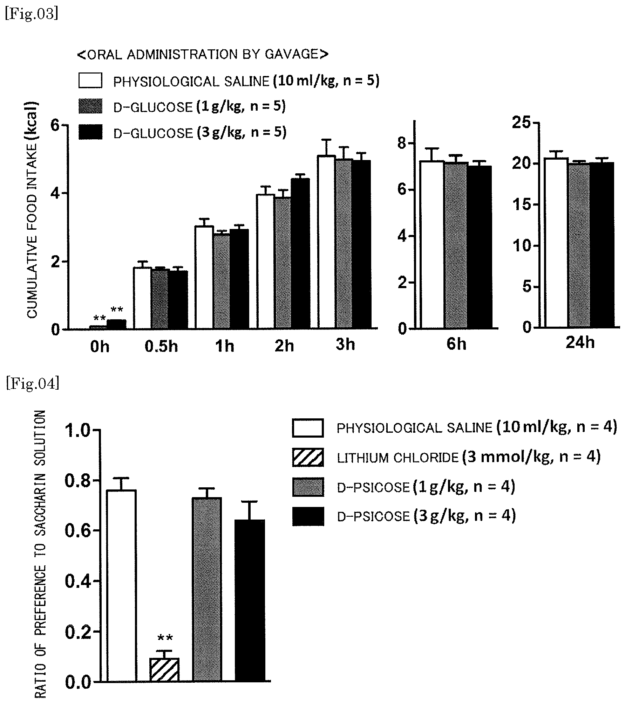

The cumulative food intake (energy (kcal)) after D-glucose (1 g/kg and 3 g/kg) was orally administered by gavage in the comparative example is shown in Fig. 3. The data at 0 hour shows the energy of D-glucose orally administered by gavage, and the cumulative food intake after 0.5 hours is expressed as the intake energy (kcal) including the energy amount of administered D-glucose. The notation of the results and the statistical analysis is the same as in Figs. 1 and 2. The oral administration by gavage of D-glucose (1 g/kg and 3 g/kg) did not affect food intake at either administration amount (Fig. 3).

Based on the above results, it was found that the oral administration by gavage of D-psicose at 1 g/kg and 3 g/kg, and the intraperitoneal administration of D-psicose at 3 g/kg have an effect of reducing food intake.

### Example 2

In order to deny that the above-described effect of reducing food intake results from discomfort or disgust accompanying the ingestion of D-psicose, a conditioned taste aversion test with respect to the oral administration of D-psicose was performed.

### <Test Method>

As the experimental animal, C57BL/6J male mice which were the same as used in the previous experiment were used. The mice reared in individual cages were given two bottles of water only for 2 hours in a period between 10:00 a.m. and 12:00 p.m. for 5 days, whereby the mice were acclimated to the water restriction schedule. On day 6, a 0.15% saccharin solution was presented for 30 minutes, and thereafter, lithium chloride (3 mmol/kg) was intraperitoneally administered, whereby the mice were made to acquire an aversion to the taste of the saccharin solution (conditioning). In the same manner, a control group in which physiological saline (10 mL/kg) was orally administered and a test group in which D-psicose (1 g/kg or 3 g/kg) was orally administered were prepared. On day 7 serving as a rest day, the above-described water restriction schedule was performed. On day 8 serving as a test day, two bottles, one bottle containing a 0.15% saccharin solution and the other bottle containing water, were simultaneously presented for 30 minutes, and the ratio of preference to the saccharin solution (the intake amount of saccharin / the total intake amount from the two bottles) was determined.

### <Test Results>

The ratio of preference to the saccharin solution after making the mice to acquire a conditioned taste aversion is shown in Fig. 4. The obtained results are expressed as a mean and a standard error. A statistical test was performed by a one-way analysis of variance, and a Dunnett's test in comparison with the control group was performed. The data was marked with ** when the P value was less than 1%.

In the physiological saline administration group, a strong preference to the saccharin solution was shown. On the other hand, in the group of administration of lithium chloride which induces visceral discomfort, a preference to the saccharin solution was significantly decreased. The oral administration by gavage of D-psicose which induces an effect of reducing food intake did not affect the preference to the saccharin solution in both cases of administration at 1 g/kg and 3 g/kg.

### <Discussion>

The oral administration by gavage of D-psicose (1 g/kg and 3 g/kg) to the mice exhibited a strong effect of reducing food intake up to 6 hours after the administration. At this time, D-psicose orally administered by gavage did not affect the taste aversion behavior, and therefore, it was found that D-psicose reduces food intake without causing disgust. Accordingly, it was suggested that the oral ingestion of D-psicose induced satiety, and food intake was reduced.

This effect of reducing food intake was not observed when administering D-glucose which is an analog of D-psicose, and therefore was an effect intrinsic to D-psicose. Further, this effect of reducing food intake was more strongly exhibited by orally administering D-psicose by gavage than by intraperitoneally administering D-psicose. Accordingly, in order to sufficiently exhibit the effect of reducing food intake by D-psicose, oral ingestion of D-psicose as a food product is considered to be an effective means. Further, as for the effective amount of D-psicose to reduce food intake in mice, it was considered that D-psicose sufficiently exhibits its effect at a low dose of 1 g/kg.

D-psicose strongly suppresses food intake by oral administration by gavage, and therefore, it was considered that D-psicose may first act on the gastrointestinal tract and reduce food intake in the end. It is known that a gastrointestinal tract hormone secreted after eating plays an important role in the formation of satiety after eating. Many of the gastrointestinal tract hormones directly act on the "vagal afferent nerves" which is a visceral sensory nerves distributed around the gastrointestinal tract, and by conveying the neural information thereof to the brain, the induction of satiety and the reduction in food intake are exhibited. Accordingly, it was considered that the activity of reducing food intake by D-psicose might be exhibited via the vagal afferent nerves.

### Example 3

A test for verifying the involvement of the vagal afferent nerves in the activity of reducing food intake induced by D-psicose was performed.

### <Test Method>

The activation of "vagal afferent nuron" and "medullary nucleus tractus solitarius", which is a projection site of the vagal afferent nerves, caused by the oral administration of D-psicose was analyzed based on the expression level of c-Fos, which is a marker of neuronal activation, using an immunostaining method. The c-Fos is a kind of immediate early gene and is an intranuclear protein whose expression level increases with the activation of a nerve and can be utilized as a marker of neuronal activation. The cell bodies of the vagal afferent neurons are all localized in the nodose ganglion, and these vagal afferent neurons are bipolar which project to the peripheral side (many internal organs such as heart, lung, gastrointestinal tract, pancreas, and liver) and the central side (medullary nucleus tractus solitarius).

As the experimental animal, C57BL/6Jmale mice were used. The mice were acclimated to the environment by preliminary rearing and handling for 1 week or more in individual cages. After fasting the mice overnight, D-psicose (1g/kg) was orally administered by gavage. At 30 minutes and 90 minutes thereafter, perfusion fixation was performed using a 4% paraformaldehyde solution, and the nodose ganglion and the brain were extracted, respectively. Each of the organs was post-fixed using a 4% paraformaldehyde solution, and a frozen section specimen was prepared. By using this frozen section specimen, the c-Fos immunostaining was performed.

### <Test Results>

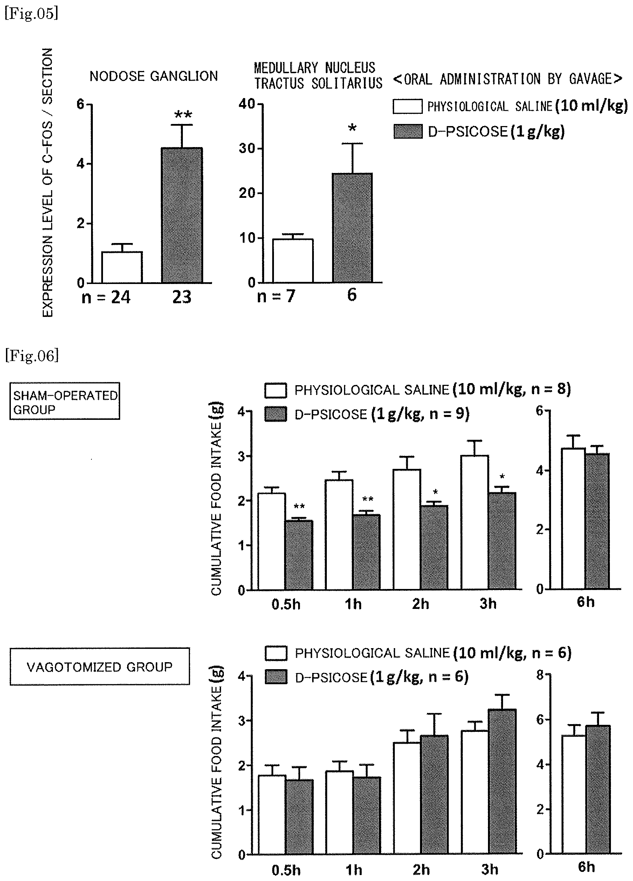

The results of the expression level of c-Fos in the nodose ganglion and in the medullary nucleus tractus solitarius in the mice after orally administering D-psicose are shown in Fig. 5. The obtained results are expressed as a mean and a standard error. A statistical test was performed using an unpaired Student's t test, and the data was marked with * when the P value was less than 5%, and the data was marked with ** when the P value was less than 1%.

As a result of this test, as compared with the physiological saline group, the oral administration by gavage of D-psicose (1 g/kg) significantly increased the expression level of c-Fos in the nodose ganglion. The oral administration by gavage of D-psicose (1 g/kg) also significantly increased the expression level of c-Fos in the medullary nucleus tractus solitarius. Accordingly, the vagal afferent nerves receiving information in the periphery directly transmits the neural information to the medullary nucleus tractus solitarius of the brain.

Based on the above-described results, it was revealed that the oral ingestion of D-psicose activates the vagal afferent nerves and the medullary nucleus tractus solitaries, which is a projection target of the vagal afferent nerves.

Therefore, in order to verify that the effect of reducing food intake by the oral ingestion of D-psicose is caused by conveying the neural information to the brain via the vagal afferent nerves, the effect of reducing food intake by D-psicose in subdiaphragmatic yagotmized mice was examined.

### <Comparative Test>

It was examined whether or not the effect of reducing food intake by D-psicose is also exhibited when it is orally administered by gavage to the mice received subdiaphragmatic vagotomy. As the experimental animal, C57BL/6J male mice were used. The mice were divided into the following two groups: a sham-operated group consisting of sham-operated mice; and a subdiaphragmatic vagotomized group consisting of subdiaphragmatic vagotomized mice, and were subjected to the operation, respectively. After a recovery period of one week, the mice were fasted overnight (for 16 hours) on the previous day of the experiment of food intake. Thereafter, each test solution was orally administered by gavage to the mice, and the intake of a liquid food (milk for babies and toddlers) after the administration was examined. As the respective test solutions administered, in both of the sham-operated group and the subdiaphragmatic vagotomized group, physiological saline (10 mL/kg) or D-psicose (1 g/kg) was orally administered by gavage. The concentration of the D-psicose solution at this time was 10%. Each test solution was orally administered by gavage to mice at 9:45 a.m., and from 10:00 a.m., the mice were allowed free access to the liquid food, and the food intake was measured over time.

### <Results of Comparative Test>

The cumulative liquid food intake after orally administering D-psicose (1 g/kg) by gavage to the subdiaphragmatic vagotomized mice and the sham-operated mice is shown in Fig. 6. The elapsed time was plotted on the abscissa, and the cumulative liquid food intake (g) at each elapsed time was plotted on the ordinate. The obtained results are expressed as a mean and a standard error. A statistical test was performed using an unpaired Student's t test, and the data was marked with * when the P value was less than 5%, and the data was marked with ** when the P value was less than 1%.

As a result, in the sham-operated group, when D-psicose (1 g/kg) was orally administered by gavage, the food intake was significantly suppressed up to 3 hours after the administration, however, in the subdiaphragmatic vagotomized group, the effect was all lost.

Based on the above-described results, it was revealed that the oral ingestion of D-psicose reduces food intake by transmitting the neural information thereof to the brain via the vagal afferent nerves.

### Example 4

A test for confirming the activity of sustaining satiety of D-psicose in humans was performed.

### <Test Method>

The test was performed by a two-group, single-blind, crossover method for seven healthy subjects who do not have smoking habit (mean age: 35.0, 4 males and 3 females, mean BMI: 21.7 kg/m²).

NPL 6 has reported that when subjects composed mainly of prediabetic adult males and females were made to ingest either of the drinks prepared to have the same degree of sweetness by adding 5 g of D-psicose or 10 mg of aspartame which is a high intensity sweetener along with breakfast in a crossover manner, and the blood glucose levels after eating were compared, a significant decrease in blood glucose level was observed in the D-psicose ingestion group.

Therefore, in this test, after fasting the subjects from 12 hours before the test day, the subjects were made to ingest either of the test solutions (150 mL) prepared to have the same degree of sweetness by adding 5 g of D-psicose or 10 mg of aspartame which is a high intensity sweetener as a control food. Incidentally, the concentration of the respective test solutions was 3.3% in the D-psicose group and 0.005% in the aspartame group. Further, the ingestion amount of D-psicose of the subjects was 0.071 to 0.096g (mean: 0.085g) per kilogram of body weight. After the ingestion, breakfast (sandwich, yogurt, and juice) (total calories: 405 kcal, proteins: 10.2%, fats: 28.4%, carbohydrates: 61.4%) was ingested in 10 minutes. During the test, the subjects were fasted except that they were allowed to free access to only prescribed water. The subjects were made to fill out the following questionnaire with respect to appetite up to 4 hours after ingestion of this breakfast, and based on these results, a change in appetite over time after eating was compared.

### <Questionnaire with respect to Appetite>

The questionnaire with respect to appetite was filled out by the evaluation using the VAS (Visual Analog Scale) method and was prepared according to NPL 11. As the questionnaire items, the following three items: satiety, hunger, and appetite were set. In each item, the subjects were made to put a mark on a line with a length of 100 mm at a position most close to the state at that moment, then, the length from the left end to the position where the mark was put was measured with a ruler and converted into a numerical value. The obtained results are expressed as a mean and a standard error. A statistical test was performed using an unpaired Student's t test, and the data was marked with * when the P value was less than 5%, the data was marked with ** when the P value was less than 1%, and the data was marked with *** when the P value was less than 0.1%.

### <Test Results>

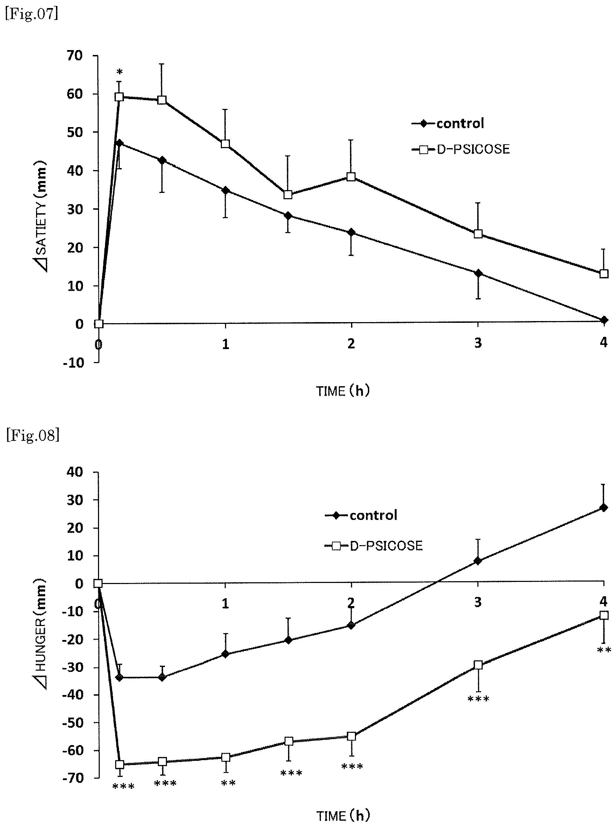

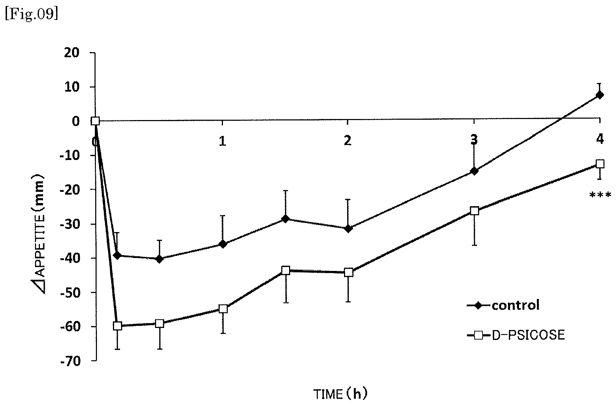

The results obtained by plotting the elapsed time (min) after ingestion of the breakfast on the abscissa, and the numerical value with respect to each item at each elapsed time on the ordinate are shown in Figs. 7 to 9. It was confirmed that in comparison with the control food, in the D-psicose ingestion group, satiety after eating was sustained, and an effect of suppressing hunger and appetite is sustained for 4 hours or more after the ingestion. In particular, hunger is significantly suppressed from immediately after the ingestion of breakfast up to after 4 hours.

### <Conclusion>

In humans, by ingesting D-psicose before eating, an effect of sustaining satiety after eating was observed.

### Industrial Applicability

According to the present invention, a novel agent for sustaining satiety and a novel method for sustaining satiety, with which satiety can be sustained although the increase in blood glucose level after eating is suppressed, can be provided. Further, even in the case of a single ingestion, the blood glucose level does not increase, and therefore, the agent and the method are useful for those who are anxious about their blood glucose level and those who are health conscious. In addition, the agent for sustaining satiety containing D-psicose of the present invention does not have side effects, and therefore can be used by incorporating it into a variety of foods and drinks or as an active ingredient of pharmaceuticals.

## Claims

1. An agent for sustaining satiety, **characterized by** comprising D-psicose as an active ingredient.

2. The agent for sustaining satiety according to claim 1, which is an agent for sustaining satiety by transmitting the oral ingestion of D-psicose to the brain via the vagal afferent nerves.

3. The agent for sustaining satiety according to claim 1, wherein D-psicose is contained such that D-psicose is ingested in an amount of 0.07 g to 3 g per kilogram of body weight with one meal.

4. The agent for sustaining satiety according to claim 1, wherein the agent is used between one hour before eating and immediately before eating.

5. The agent for sustaining satiety according to claim 1, wherein the agent is used for an individual who needs suppression of the increase in blood glucose level after eating or an obese individual.

6. The agent for sustaining satiety according to claim 1, wherein the agent is used for producing a diet food or drink.

7. A method for sustaining satiety, **characterized by** comprising a step of making a subject to ingest D-psicose or administering D-psicose to a subject.

8. The method for sustaining satiety according to claim 7,
wherein the step of ingesting or administering D-psicose comprises ingesting or administering D-psicose in an amount of 0.07 g to 3.0 g per kilogram of body weight with one meal.

9. The method for sustaining satiety according to claim 7,
wherein the step of ingesting or administering D-psicose is provided between one hour before eating and immediately before eating.

10. The method for sustaining satiety according to claim 7,
wherein the step of ingesting or administering D-psicose is provided at every meal.

11. The method for sustaining satiety according to claim 7, wherein the step of ingesting or administering D-psicose is provided once daily before breakfast.

12. The method for sustaining satiety according to claim 7,
wherein the step of ingesting or administering D-psicose is performed through oral ingestion or oral administration of D-psicose, and the oral ingestion or oral administration thereof is transmitted to the brain via the vagal afferent nerves, whereby satiety is sustained.

13. The method for sustaining satiety according to claim 7,
wherein the subject is an individual who needs suppression of the increase in blood glucose level after eating or an obese individual.
